(19) Europäisches Patentamt
European Patent Office
Office européen des brevets

(11) EP 3 615 536 B1

(12) # EUROPEAN PATENT SPECIFICATION

(45) Date of publication and mention of the grant of the patent:
**29.05.2024 Bulletin 2024/22**

(21) Application number: **18721752.6**

(22) Date of filing: **27.04.2018**

(51) International Patent Classification (IPC):
***C07D 487/04*** (2006.01) ***C07D 487/22*** (2006.01)
***A61P 7/00*** (2006.01) ***A61P 9/10*** (2006.01)
***A61P 9/12*** (2006.01)

(52) Cooperative Patent Classification (CPC):
**C07D 487/22; A61P 7/00; A61P 9/10; A61P 9/12; C07D 487/04**

(86) International application number:
**PCT/EP2018/060963**

(87) International publication number:
**WO 2018/197705 (01.11.2018 Gazette 2018/44)**

(54) **HYDROXY SUBSTITUTED CUCURBITURIL DERIVATIVES AS OXYGEN CARRIERS**

HYDROXY-SUBSTITUIERTE CUCURBITURILDERIVATE ALS SAUERSTOFFTRÄGER

DÉRIVÉS DE CUCURBITURIL SUBSTITUES PAR HYDROXY EN TANT QUE TRANSPORTEURS D'OXYGÈNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2017 EP 17305480**

(43) Date of publication of application:
**04.03.2020 Bulletin 2020/10**

(73) Proprietor: **Prieur, Benoit**
**75005 Paris (FR)**

(72) Inventors:
- **HECK, Marie-Pierre**
**78180 Montigny le Bretonneux (FR)**
- **HUBER, Gaspard**
**91300 Massy (FR)**
- **PRIEUR, Benoit**
**91300 Massy (FR)**

(74) Representative: **Regimbeau**
**20, rue de Chazelles**
**75847 Paris Cedex 17 (FR)**

(56) References cited:
**WO-A1-00/68232** **WO-A1-03/055888**
**JP-A- 2003 212 877** **US-A1- 2003 140 787**

- **MIYAHARA Y ET AL: ""Molecular" Molecular Sieves: Lid-Free Decamethylcucurbit[5]uril Absorbes and Desorbs Gases Selectively", ANGEWANDTE CH, WILEY - V C H VERLAG GMBH & CO. KGAA, DE, vol. 114, no. 16, 16 August 2002 (2002-08-16), pages 3146-3149, XP008120926, ISSN: 0044-8249, DOI: 10.1002/1521-3757(20020816)114:16<3146::AID-ANGE3146>3.0.CO;2-U [retrieved on 2002-08-22]**
- **MOCK ET AL.: "Structure and Selectivity in Host-Guest Complexes of Cucurbituril", JOURNAL OF ORGANIC CHEMISTRY, vol. 51, 1986, pages 4440-4446, XP002771320,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 615 536 B1

## Description

The present invention relates to cucurbituril derivatives and their therapeutic and non-therapeutic applications as physiological gases carriers.

Cucurbituril is a cyclic oligomer made of six glycoluril monomers ($C_4H_2N_4O_2$) linked by methylene bridges ($-CH_2-$), which was first synthetized in 1905 by Behrend and coworkers [Behrend et al., Justus Liebigs Ann. Chem. Soc., 1905, 339, 1].

The name cucurbituril is derived from the resemblance of this molecule with a pumpkin of the family of *Cucurbitaceae,* as noticed by Mock and coworkers who described its structure [Mock et al., J. Am. Chem. Soc., 1981, 103, 7367-7368].

Said structure corresponds to the following formula (CB):

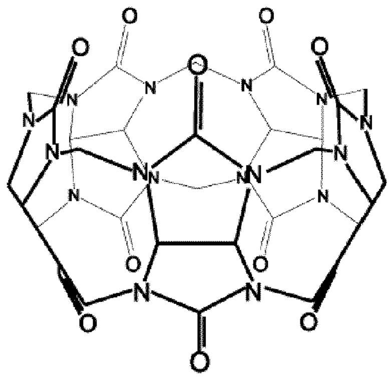

(CB)

By extension, cucurbit[n]urils, abbreviated CB[n], refer to macrocyclic molecules made of n glycoluril units bounded together via methylene bridges.

Their distinctive internal and rigid cavity enables cucurbit[n]urils to host miscellaneous compounds, such as peptides, saccharides, dyes, hydrocarbons, drugs or even proteins with a remarkable selectivity in binding, hence paving the way for numerous applications in a broad spectrum of areas, notably catalysis, biology, medicine, material or environmental sciences.

This has led to an ever-increasing interest for this family of compounds and the synthesis of new homologues and derivatives [Nau et al., Chem. Soc. Rev., 2015, 44, 394-418].

A method for producing cucurbit[n]urils formed by glycoluril monomers being optionally substituted on the carbon atoms that are shared by the fused imidazolidine rings has thus been patented by Day et al. [US 6,793,839], and a method for binding a gas or volatile compound in a cucurbit[n]uril has been disclosed afterwards by the same inventors [US 6,869,466].

Hydroxycucurbiturils are described in WO03/055888.

The ability to fix, carry and release physiological gases is of tremendous interest. In particular, countless industrial and therapeutic applications are conceivable with regard to oxygen carriers.

For instance, the biotechnology company Hemarina SA is dedicated to the development of uses of marine extracellular hemoglobin-based oxygen carriers as blood substitute and in the fields of organ preservation, wound dressing and cell-culture [WO 2010/128159].

The field of blood substitutes in particular has drawn a lot of attention in recent years, due to insufficient supplies of blood.

Indeed, in spite of the collection of around 108 million units of donated blood every year worldwide, many patients requiring transfusion do not have timely access to safe blood and blood products. The transfusion is commonly used for supportive care in cardiovascular surgery, transplant surgery, massive trauma, and therapy for solid and haematological malignancies. Besides, while all donated blood should always be screened for HIV, hepatitis B, hepatitis C and syphilis prior to transfusion, many low and middle-income countries are not able to screen all donated blood for one or more of these infections, because of irregular supply of test kits, staff shortages, poor quality test kits, or lack of basic quality in laboratories.

The development of safe blood substitutes could thus tackle these issues.

Even though the marine extracellular hemoglobin could be an adequate substitute for human hemoglobin, the fact still remains that it is unlikely that it can be produced in sufficient quantities to meet the needs for blood intended for transfusion, when one takes into consideration that one liter of human blood contains approximately 140 g of hemoglobin. Besides, the use of a biological product involves a risk of a contamination since it might contain a virus.

Therefore, there is a need for new oxygen and more generally physiological gases carriers.

The applicant has recently discovered that a specific subclass of cucurbit[n]urils exhibits a remarkably strong affinity for oxygen.

The invention is set out in the appended set of claims.

The present invention thus relates to a compound of the following general formula (I):

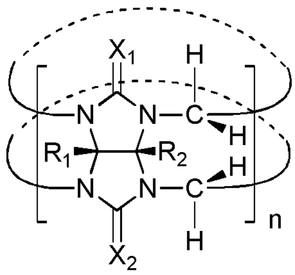

(I)

or a salt, a solvate or a stereoisomer thereof, wherein:

- n is equal to 5,
- $X_1$ and $X_2$ represent, independently of each other, an oxygen atom, and
- $R_1$ and $R_2$ each represent, independently of each other, a $OR_3$ group wherein
- $R_3$ each represent, independently of each other, a hydrogen atom,

for use as a physiological gas carrier, wherein said gas is $O_2$.

Within the meaning of the present disclosure the term "physiological gas" refers to a gas involved in human body functions, such as $O_2$, $N_2$, NO, $NO_2$, $H_2$, He or $CO_2$, in particular $O_2$, NO or $CO_2$. According to the invention, said physiological gas is $O_2$.

In the context of the present invention, a salt can be:

(1) an acid addition salt formed with an inorganic acid such as hydrochloric, hydrobromic, sulfuric, nitric and phosphoric acid and the like; or formed with an organic acid such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic and trifluoroacetic acid and the like, or

(2) a salt formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate, sodium hydroxide and the like.

In the context of the present invention, solvates of the compounds for use of the present invention include conventional solvates such as those formed during the last step of the preparation of the compounds of the invention due to the presence of solvents. As an example, mention may be made of solvates due to the presence of water (these solvates are also called hydrates) or ethanol.

Within the meaning of this invention, "stereoisomers" is intended to designate diastereoisomers or enantiomers. These are therefore optical isomers. The optical isomers result from the different position in space of substituents or lone pair of electrons on an atom (such as a carbon atom) comprising four different substituents (including potentially a lone pair of electron). This atom thus represents a chiral or asymmetric center. Optical isomers which are not mirror images of one another are designated as "diastereoisomers," and optical isomers which are non-superimposable mirror images are designated as "enantiomers".

An equimolar mixture of two enantiomers of a chiral compound is designated as racemate or racemic mixture.

The term "halogen" as used in the present invention refers to an atom of fluorine, bromine, chlorine or iodine.

The term "($C_1$-$C_6$)alkyl" as used in the present invention refers to a saturated, linear or branched hydrocarbon chain comprising from 1 to 6 carbon atoms, including, but not limited to, methyl, ethyl, n-propyl, isopropyl, n-butyl, isobutyl, sec-butyl, tert-butyl, n-pentyl, n-hexyl and the like.

The compound for use according to the invention may be represented by one of the following equivalent schematic structures (S1) and (S2), in which i is equal to 0 or 1:

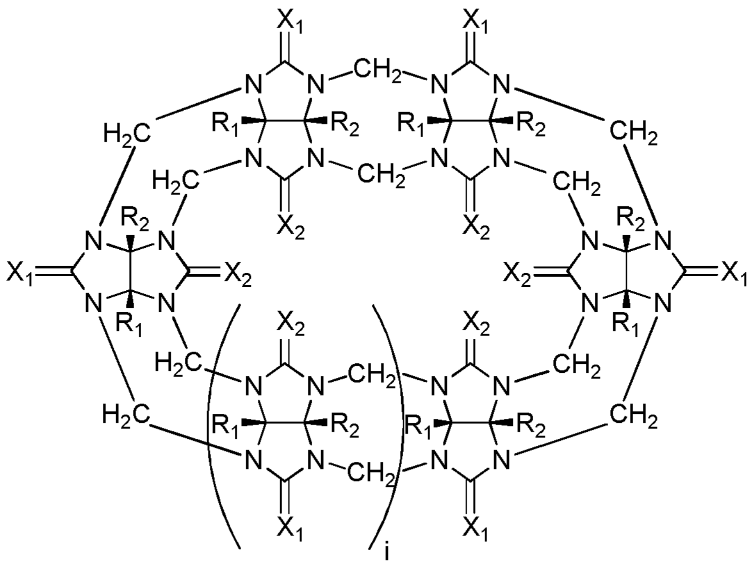

(S1)

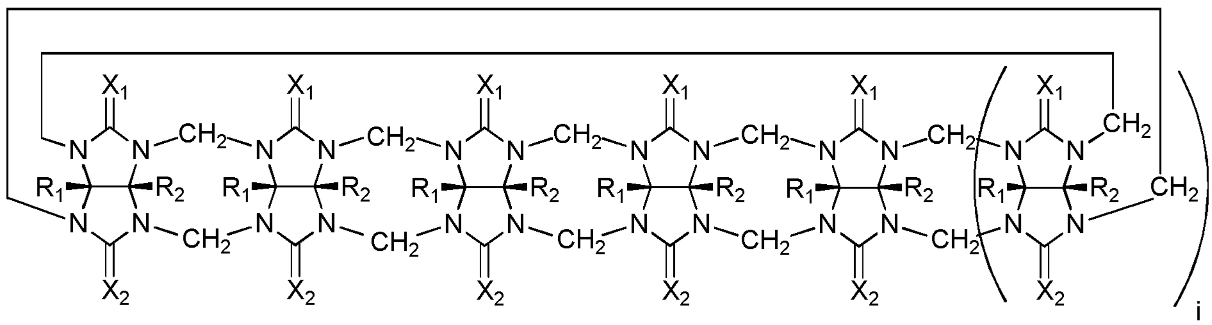

(S2)

Indeed, a compound for use according to the invention is a cyclic oligomer made of n substituted glycoluril monomers of the following formula (GU) linked by methylene bridges ($-CH_2-$), n being equal to 5:

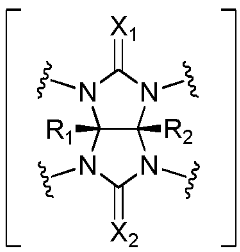

(GU)

or a salt, a solvate or a stereoisomer thereof.

The compound for use according to the invention is $CB[5]OH_{10}$, which thus corresponds to the following formula:

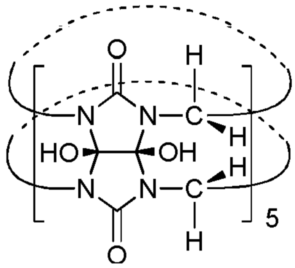

or a salt or a solvate thereof.

In another particular embodiment, one or several hydrophilic polymeric side-chains are grafted onto a compound for use according to the invention as defined above, each hydrophilic polymeric side-chain being covalently bound to the molecule via a $R_1$ or $R_2$ group and being a linear or branched chain of water-soluble monomers, wherein said polymer is a polyol or a saccharide.

The term "polyol" as used in the present invention refers to a $(C_1-C_6)$alkyl as defined above which further contains at least 2, preferably 2 or 3 hydroxyl groups, including, but not limited to, glycerol, ethyleneglycol and the like.

The term "saccharide" as used in the present invention refers to erythrose, threose, ribose, arabinose, xylose, lyxose, allose, altrose, glucose, mannose, gulose, idose, galactose, talose, erythrulose, ribulose, xylulose, psicose, fructose, sorbose or tagatose, in D or L form, notably glucose, optionally substituted by one or more $(C_1-C_6)$alkyl groups, such as methyl, ethyl or n-propyl groups.

In still another particular embodiment, a compound for use according to the invention is covalently linked to one or several hydrophilic polymeric side-chains, such as polyethyleneglycol (PEG) or hydroxyethyl starch (HES).

The compound as defined above is for use as a $O_2$ carrier.

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

In another embodiment, the compound as defined above is used as a hemoglobin substitute.

In the context of the present invention, the term "hemoglobin substitute" refers to a compound capable of replacing the hemoglobin present in the red blood corpuscles and capable of performing its functions as gas carrier, especially with respect to oxygen and carbon dioxide. This substitute also has to supply oxygen to the tissues, where it becomes charged with $CO_2$, to release it at the exchange surfaces, namely the lungs.

In another embodiment, the compound as defined above is used for the treatment of ischemia.

In the context of the present invention, the term "ischemia" is meant to refer to an insufficiency in blood supply to tissues, leading notably to a shortage of oxygen.

It can be in particular renal ischemia, cardiac ischemia (aka myocardial infraction or acute myocardial infraction, commonly named heart attack), ischemic colitis, mesenteric ischemia, acute or chronic brain ischemia, acute limb ischemia, or cutaneous ischemia.

The present invention also relates to a method for treating ischemia, comprising the administration to a person in need thereof of an effective dose of a compound as defined above.

In another embodiment, the compound as defined above is for use as a $O_2$ and/or NO carrier intended for the regulation of arterial blood pressure.

In still another embodiment, the compound as defined above is for use as a $N_2$ carrier intended for removing said gas from the blood of a patient following a diving accident.

In yet another embodiment, the compound as defined above is for use in anti-ageing, skin protection or skin regeneration.

The present invention is also directed to a complex of a compound as defined above comprising a gas encapsulated in its inner cavity, said gas is $O_2$, said compound is $CB[5]OH_{10}$.

The present invention also relates to a complex as defined above according to the invention for use in the treatment of ischemia.

The present invention also relates to a method for treating ischemia, comprising the administration to a person in need thereof of an effective dose of a complex as defined above.

The present invention also relates to a complex of the invention for use for the regulation of arterial blood pressure.

The present invention also relates to a complex of the invention for use in anti-ageing, skin protection or skin regeneration.

The present invention also relates to a composition consisting in a physiologically acceptable medium comprising a compound and/or a complex as defined above for use as a blood substitute, notably human blood substitute.

The term "physiologically acceptable medium" refers to a medium that is generally safe, nontoxic and neither biologically nor otherwise undesirable and which is acceptable for veterinary as well as human therapeutical use.

It can further contain salts, such as NaCl, $CaCl_2$, $MgCl_2$, $MgSO_4$, KCl, sodium gluconate and/or sodium acetate.

The composition according to the invention can be formulated in particular for oral administration or for administration by injection, said composition being intended for mammals, including humans.

The active ingredient can be administered in unit dosage forms, mixed with standard pharmaceutical excipients, to animals or to human beings.

The suitable oral unit dosage forms include tablets, capsules, powders, granules and oral solutions or suspensions.

When a solid composition is prepared in tablet form, the principal active ingredient is mixed with a pharmaceutical vehicle such as gelatin, starch, lactose, magnesium stearate, talc, gum arabic or analogues. The tablets can be coated with sucrose or other suitable materials or they can be treated so that they have a prolonged or delayed activity and that they continuously release a predetermined quantity of active ingredient.

A capsule preparation is obtained by mixing the active ingredient with a diluent and pouring the mixture obtained into soft or hard capsules.

A preparation in syrup or elixir form can contain the active ingredient together with a sweetener, an antiseptic, as well as a flavor enhancer and a suitable dye.

The water-dispersible powders or granules can contain the active ingredient mixed with dispersants or wetting agents, or suspending agents, as well as with flavor enhancers or sweeteners.

For administration by injection, one uses aqueous suspensions, isotonic saline solutions or sterile solutions for injection that contain pharmacologically compatible dispersants and/or wetting agents.

The active ingredient can be also formulated in microcapsule form, optionally with one or more additive excipients.

The present invention also relates to a composition as defined above for use in the treatment of ischemia.

The present invention also relates to a method for treating ischemia, comprising the administration to a person in need thereof of an effective dose of a composition as defined above.

The present invention also relates to a composition as defined above for anti-ageing, skin protection or skin regeneration.

The present invention is also directed to a non-therapeutic use of a compound of the following formula:

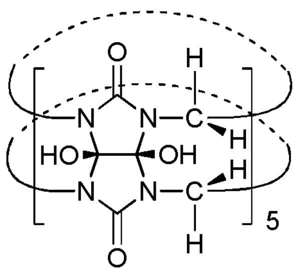

or a salt, a solvate or a stereoisomer thereof,
for binding, carrying and/or releasing a gas, wherein said gas is $O_2$.

In another embodiment, said compound is covalently linked to one or several hydrophilic polymeric side-chains, such as polyethyleneglycol (PEG) or hydroxyethyl starch (HES).

The use according to the invention is the use of $CB[5]OH_{10}$ for binding, carrying and/or releasing $O_2$.

The present invention also relates to the use, in particular a non-therapeutic use of a compound as defined above a complex according to the invention for the preservation and/or protection of biological materials such as cells, tissues, body fluids and organs in vitro, and of microorganisms, advantageously as a medical device.

In the context of the present invention, "in vitro" means outside of the organism from which the biological material derives.

The present invention relates also to a method of preservation and/or protection of biological materials and of microorganisms by placing said biological materials in a medium containing a compound or a complex as defined above.

The term "preservation" of a biological material or of a microorganism as used in the present invention refers to the fact to maintain the state (notably the structure and function) of the biological material or microorganism as it already exists or to prevent or limit the degradation of this state.

The term "protection" of a biological material or of a microorganism as used in the present invention refers to the fact that the biological material or microorganism is protected against an internal or external aggression, such as a stress, for ex. an oxidative stress (for ex. UV), a change of temperature, a change of pH, a chemical or bacterial

contamination, starvation conditions, etc.

In particular, a biological material or a microorganism can be protected when placed at a temperature below 37°C, such as below 0°C, notably in conditions of cryopreservation in particular for biological materials such as human organs, tissues (e.g. for transplant), body fluids or cells.

The cryopreservation of a biological material or a microorganism implies to cool to sub-zero temperatures the biological material or microorganism, and notably at a temperature of about -196°C by using liquid nitrogen.

The biological material can be in particular cells, tissues, body fluids or organs.

The microorganism can be in particular a prokaryotic or eukaryotic microorganism, being notably unicellular or pluricellular. The microorganism can be notably chosen among bacteria, fungi, including yeasts, algae, viruses, including phages, microparasites (also called parasitic microorganisms) and protozoa.

In the context of the present invention, a "medical device" refers to any product which is put in contact with organs, tissues, cells or products from the human or animal body origin during their conservation, their preparation, their transformation, their packaging or their transport prior to their therapeutic use in humans. A medical device according to the present invention can also be any product coming into contact with embryos in the context of an activity of medically assisted procreation. In particular, this category of products includes graft preservation media (tissues, organs), the media used in the context of in vitro fertilization, or media used during the preparation of cell therapy products.

In particular, the present invention is directed to the use of a compound or a complex as defined above, for use in medium for preserving organs, biological tissue, or living cells, preferably for preserving organs such as for example liver or kidney.

Said compound or complex can thus be used in the case of a graft as a supplementary therapeutic product for preserving cells, tissues or organs between the sampling on a donor and the graft on a receiver.

The present invention is also directed to a culture, storage and/or preservation medium comprising at least one compound or one complex as defined above.

The present invention relates also to the use of a compound or a complex as defined above as an adjuvant in a culture, storage and/or preservation medium.

The culture, storage and/or preservation medium is intended for the culture, storage and/or preservation of a biological material or of a microorganism. The biological material will be more particularly cells or tissues in the case of a culture medium.

The present invention relates also to a culture, storage and/or preservation medium comprising at least one compound or one complex as defined above.

The culture, storage and/or preservation medium can be liquid or in the form of a gel. It contains thus water. However, the medium can be in a dehydrated form which can be rehydrated by water addition.

It can contain one or several components of the group consisting of co-solvents (e.g. dimethylsulfoxyde (DMSO)), salts (for ex. NaCl, $MgCl_2$, $ZnCl_2$, $MnCl_2$, $CuCl_2$, $K_2PO_4$, $KH_2PO_4$, $K_2HPO_4$, $Na_2S_2O_3$, $K_2SO_4$, $MgSO_4$, $KNO_3$, $Ca(NO_3)_2$, $Na_2CO_3$, $NaHCO_3$, etc.), carbon sources such as carbohydrates (for ex. glucose, lactose or sucrose) or polyols (for ex mannitol or glycerol), vitamins (for ex. vitamins B1, B2, B6, B12, B3, B5, B9, B7, C, A, D, E and K), nitrogen and amino acid sources (for ex. peptones, beef or yeast extract, serum, etc.), growth factors (for ex. insulin, transferrin, fibonectin, albumin), differentiating factors, antibiotics and antimycotics (also called antibacterial and antifungal agents - e.g. actinomycin D, amphotericin B, ampicillin, carbenicillin, cefotaxime, fosmidomycin, gentamicin, kanamycin, neomycin, streptomycin, penicillin, polymixin B), hormones, cytokines and trace elements.

Other additives can be present such as indicators (of pH for example), inhibitors, etc.

When it is in the form of a gel, the culture medium can further comprise a gelling agent such as agar, gelatine, silica gel, etc.

The present invention relates also to a use, advantageously non-therapeutic, preferably cosmetic use of a compound as defined above and/or a complex according to the invention for anti-ageing, skin protection or skin regeneration.

The present invention relates also to a cosmetic method for anti-ageing, skin protection or skin regeneration by applying a compound as defined above and/or a complex according to the invention to the skin.

The present invention relates also to a method for anti-ageing, skin protection or skin regeneration by applying to the skin of a person in need thereof of an affective amount of a compound as defined above as and/or a complex according to the invention.

In such use or method, the compound or the complex can be applied topically on the skin.

The present invention relates also to a cosmetic composition comprising at least one compound as defined above and/or a complex according to the invention and at least one cosmetically acceptable excipient.

The composition of the invention can also comprise one or more additive(s), such as antimicrobial agents, antioxidants, dermatologically active agents, emollients, other humectants, other thickening agents, fragrances, preservatives, pigments or colorants or opacifiers.

These additional additives will be incorporated in limited weight amounts so as not to interfere with the viscosity,

moisturizing and rinse-off characteristics of the base composition. Such additives are conventional to those of skill in the art.

Examples of these additives are listed below as well as in the International Cosmetic Ingredient Dictionary and Handbook, eds. Wenninger and McEwen (The Cosmetic, Toiletry, and Fragrance Assoc., Washington, D.C., 7@th Edition, 1997) (hereinafter "ICT Handbook").

Antimicrobial agents can be used when the composition is to be applied to skin prone to microbial infection, e.g., by bacteria, fungal, or protozoa. Examples of such agents include benzyl alcohol, chlorobutanol, phenylethyl alcohol, phenylmercuric acetate, potassium sorbate, sorbic acid, benzoic acid, phenoxyethanol, butylparaben, ethylparaben, methylparaben, propylparaben, and sodium benzoate, and in particular methylparaben. Other antimicrobial agents are listed on page 1612 of the ICT handbook.

Antioxidants can be used to protect ingredients of the composition from oxidizing agents that are included within or come in contact with the composition. Examples of antioxidants include ascorbyl palmitate, butytlated hydroxyanisole, butylated hydroxytoluene, potassium propyl gallate, octyl gallate, dodecyl gallate, phenyl-$\alpha$-napthyl-amine, and tocopherols such as $\alpha$-tocopherol. Other antioxidants are listed on pages 1612-13 of the ICT Handbook.

Dermatologically active agents include agents for treating wound healing, inflammation, acne, psoriasis, cutaneous aging, skin cancer, impetigo, herpes, chickenpox, dermatitis, pain, itching or skin irritation. Examples of such dermatologically active agents include hydrocortisone, dexamethesone, panthenol, phenol, tetracycline hydrochloride, yeast, hexylresorcinol, lamin, kinetin, betamethasone, triamcinolone, fluocinolone, methylprednisolone, retinoids such as retinol and retinoic acid, dapsone, sulfasalazine, resorcinol, salicylic acid, benzoyl peroxide, erythromycin-benzoyl peroxide, erythromycin, clindamycin, mupirocin, griseofulvin, azoles such as miconazole, econozole, itraconazole, fluconazole, and ketoconazole, ciclopirox, allylamines such as naftifine and terfinafine, acyclovir, famciclovir, valacyclovir, benzocaine, lidocaine, dibucaine, pramoxine hydrochloride, methyl salicylate, camphor, menthol, resocinol, and vitamins such as tocopherol, and tocopherol acetate.

Emollients are agents that soften and smooth the skin. Examples of emollients include oils and waxes such as microcrystaline wax, polyethylene, triglyceride esters such as those of castor oil, cocoa butter, safflower oil, corn oil, olive oil, cod liver oil, almond oil, palm oil, squalene, and soybean oil, acetylated monoglycerides, ethoxylated glycerides, fatty acids, alkyl esters of fatty acids, alkenyl esters of fatty acids, fatty alcohols, fatty alcohol ethers, ether-esters, lanolin and derivatives of lanolin, polyhydric alcohol esters, wax esters such as beeswax, vegetable waxes, phospholids, and sterols, isopropyl palmitate or glyceryl stearate, and in particular almond oil or fatty alcohols such as cetyl, stearyl and/or myristyl alcohols. Other emollients are listed on pages 1656-61 of the ICT handbook.

Siloxanes are particularly preferred emollient. Siloxanes that may be used in the present invention include, but are not limited to, dimethicone, cyclomethicone, phenyl trimethicone, phenyl dimethicone, cetyl dimethicone, stearyl dimethicone, amodimethicone, $C_{30-45}$ alkyl dimethicone, $C_{30-45}$ Alkyl Methicone, Cetearyl methicone, dimethicone copolyol, cyclopentasiloxane, cyclohexasiloxane or any combinations thereof. In particular, amodimethicone could be used as emollient in the present invention.

Additional thickening agents could be in particular fatty alcohols such as cetyl, stearyl and/or myristyl alcohols.

Examples of fragrances or perfume include peppermint, rose oil, rose water, aloe vera, clove oil, menthol, camphor, eucalyptus oil, and other plant extracts. To eliminate certain odours from compositions, masking agents may be used. Other fragrances and masking agents are listed on pages 1639-40 of the ICT Handbook.

Preservatives can be used to protect the composition from degradation. Examples of preservatives include phenoxyethanol, methylparaben, benzalkonium chloride, benzethonium chloride, propyl paraben, benzoic acid, benzyl alcohol, and mixtures thereof such as liquipar oil. In particular, it can be phenoxyethanol, methylparaben or a mixture thereof. Other preservatives are listed on pages 1654-55 of the ICT Handbook. However, the composition of the present invention can be preservative free.

Pigments or colorants are used to modify the color of the composition, such as to obtain a white composition. It can be in particular titanium dioxide.

Opacifiers, such as titanium oxide, are used in clear or transparent composition in order to render it opaque. The present invention can thus be clear or opaque according to the use or not of an opacifier.

The cosmetic composition according to the invention can be formulated in particular for a topical administration. It can thus be a lotion, a foam, a gel, a dispersion, a suspension, a spray, a serum, a cream, an emulsion, a body milk, a shampoo, or also a mask.

The cosmetic composition is intended in particular for anti-ageing, skin protection or skin regeneration.

The present invention also relates to the use of a compound as defined above for catalysing a chemical reaction.

The present invention relates also to the use of a compound as defined above for releasing $O_2$ in a medium intended for aquaculture.

In a preferred embodiment, said medium is an aquarium.

The present invention also relates to the use of a compound as defined above for releasing $O_2$ in a medium intended for agriculture, particularly for the growth of a vegetable species.

In a preferred embodiment, said medium is deprived of soil, and comprises a liquid containing nutriments that contribute to the growth of the vegetable species.

The present invention relates also to the use of a compound as defined above for releasing $O_2$ in a medium that contains aerobic decontaminating microorganisms.

## FIGURES

- Figures 1a and 1b represent the $^1H$ NMR spectra obtained at 20°C for CB[5]$OH_{10}$, depending on whether $O_2$ and NaCl are present or not:
  - SP1: without NaCl and $O_2$;
  - SP2: with $O_2$ under 1 bar, no NaCl ; and
  - SP3: with NaCl and $O_2$ at the pressure of 1 bar.
- Figure 2 displays the $^1H$ NMR spectra obtained at 37°C for CB[5]$OH_{10}$ in function of the oxygen partial pressure, said spectra being focused on the $H_{ax}$ signal.
- Figure 3 represents the $R_1$ measured for $H_{ax}$ at 37°C in function of the oxygen partial pressure.
- Figures 4a and 4b represent the $^1H$ NMR spectra SP1 and SP2 obtained at 20°C for CB, depending on whether $O_2$ is present or not.
- Figures 5a and 5b represent the $^1H$ NMR spectra obtained at 20°C for CB[5]$Me_{10}$, depending on whether $O_2$ and NaCl are present or not:
  - SP1: without NaCl and $O_2$;
  - SP2: with $O_2$ under 1 bar, no NaCl; and
  - SP3: with NaCl and $O_2$ at the pressure of 1 bar.

The examples that follow illustrate the invention without limiting its scope in any way.

## EXAMPLES

The following abbreviations have been used:

| | |
|---|---|
| ax. | : axial |
| ca. | : *circa* |
| eq. | : equatorial |
| Me | : Methyl ($CH_3$) |
| NMR | : Nuclear Magnetic Resonance |
| TSP-$d_4$ | : 3-(Trimethylsilyl)propionic-2,2,3,3-$d_4$ acid sodium salt |

## I - Materials and methods

### I-1. Studied compounds

The ability of CB[5], CB[5]$Me_{10}$ and CB[5]$OH_{10}$ to fix $O_2$ has been studied.

Said compounds are of the following formula (IV), wherein $R_1$ corresponds to H, $CH_3$ and OH respectively.

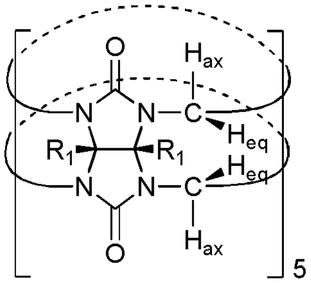

(IV)

**I-2. Synthesis**

- **$CB[5]OH_{10}$** was synthetized following the method described in the literature (J. Am. Chem. Soc. 2003, 125, 10186) with a minor modification when treating the manipulation: the volume of solvent is half reduced under reduced pressure and precipitated with acetone. The filtrate is concentrated and agitated during 12 hours with a Amberlyst A21 resin (Flucka 20-50 mesh), and then filtrated on a sinter filter (P4 Porosity) and concentrated under vacuum to lead to $CB[5]OH_{10}$.

- **$CB[5]Me_{10}$** was synthetized following the method described in the literature (Angew. Chem. Int. Ed. 1992, 31, 1475).

- **CB[5]** was bought from Sigma Aldrich.

**I-3. $^1$H NMR**

- *Samples preparation*:

- $CB[5]OH_{10}$: 0.319 mM solution in D20 (and NaCl when mentioned);
- CB[5]: 0.501 mM solution in D20 (and NaCl when mentioned); and
- $CB[5]Me_{10}$: 0.472 mM solution in D20 (and NaCl when mentioned).

The concentrations were measured in separate samples by adding a known volume to a known solution of TSP-$d_4$.

Samples containing $O_2$ to the pressure of ca. 1 bar where prepared by addition of pure $O_2$ at a pressure slightly higher than atmospheric pressure using a syringe, then equilibration of the solution and quick release towards the ambient air.

In fact, the experience is conducted on a 156 m altitude and the medium pressure is 0.982 bar.

An experience involving a sample containing $O_2$ to the pressure of 5 bar has been conducted: $O_2$ has been added by condensation of a known quantity of $O_2$ in a previously degassed tube, using a bath of liquid nitrogen.

- *Measurement of $T_1$*:

The longitudinal relaxation time $T_1$ is measured by the standard inversion-recovery method (180° -t-90° acq.), followed by phasing of the spectrum and a baseline correction in Topspin®. The data is then exported to Excel. The signals are integrated and the intensity is adjusted according to the inversion delay based on a 3-parameter model:

$$S = S_0\left(1 - 2A \times e^{-R_1 t}\right)$$

wherein the A factor takes into account the imperfect inversion of the magnetization, and $R_1$ is the relaxation rate, reverse of $T_1$.

- *Adjustment of the complexing constant K:*

The adjustment of the complexing constant K is obtained by adjusting the 1:1 complexation model as follows:

$$R_1 = \frac{R_{1\,empty}[compound]_{empty} + R_{1\,complex}[complex]}{[compound]_{total}}$$

By definition:

$$K = \frac{[complex]}{[O_2][compound]_{empty}}$$

with:

$$[complex] + [compound]_{empty} - [compound]_{total}$$

thus, leading to:

$$R_1 = R_{1\,complex} \quad \frac{1}{1 + sKP_{O_2}}\left(R_{1\,empty} \quad R_{1\,complex}\right)$$

wherein:

$R_{1\,empty}$, which corresponds to $R_1$ of the nucleus considered in the absence of oxygen, is obtained with a fairly good accuracy (10%).

$R_{1\,complex}$, which corresponds to $R_1$ of the nucleus when a complex of the tested compound and $O_2$ encapsulated in its inner cavity (or cage) is formed, is an adjustable parameter. It is not known precisely because, in view of the results, a pressure much higher than the experimental pressures (maximum 5 bar) would be required to saturate the cages almost completely.

s, which is the solubility of the oxygen in a 9 g/l NaCl solution at 37°C, is obtained by intrapolation of tabulated data. Indeed, the concentration of $O_2$ in solution is proportional to $P_{O2}$, the $O_2$ pressure above the solution: Henry's law is very well verified in the experimental pressure range.

## II - Results

### II-1. CB[5]OH$_{10}$

- *Attribution:*

When considering the $^1$H NMR spectra obtained at 20°C displayed on figures 1a and 1b, the attribution is obvious by massifs. Indeed, the 10 methylenes are equivalent. $H_{ax}$ (see formula (IV)) resonates at ca. 5.40 ppm, and $H_{eq}$. at ca. 4.58 ppm.

Besides, each signal is composed of 2 doublets:

- one depends on the presence or not of $O_2$ (circles), whose paramagnetism has a considerable influence on the NMR signals nearby; the linewidth and the value of $T_1$ vary greatly.
- for the other one (black circles), the NMR characteristics are invariable by the addition of $O_2$. It is therefore hypothesized that the cage-molecules corresponding to the black circles contain a molecule of high affinity, which might be $N_2$ or a solvent. The encapsulation of said molecule could be prevented by working under an higher oxygen partial pressure, by using an oxygen / helium mixture, or by removing said molecule by purification.

The circles correspond to a weighted average between "empty" molecules (i.e. containing no molecules other

than solvent), and complexes of CB[5]$OH_{10}$ and $O_2$. Indeed, as previously stated, a pressure much higher than the experimental pressures (maximum 5 bar) would be required to saturate the cages almost completely.

The fact that the encapsulation of $O_2$ is favoured under higher oxygen partial pressure is evidenced by figure 2, which represents the $^1H$ NMR spectra obtained at 37°C in function of said pressure of samples containing NaCl at physiological concentrations.

Therefore, $CB_5OH_{10}$ significantly encapsulates $O_2$ at 37°C even in the presence of NaCl at physiological concentrations.

- *Determination* of *K via $R_1$:*

• With respect to the $H_{ax}$ signal of interest:

$T_{1empty}$, measured using the degassed sample, is equal to 2.1 $s^{-1}$.
$T_1$, and hence $R_1$, was measured for various pressures of oxygen.
The results obtained are displayed on figure 3.
The adjustment gives K = 3000 $M^{-1}$. The sum of the square of the deviations to the model is doubled for K = 1170 and 6380 $M^{-1}$, giving an estimate of the measurement error.

• $R_1$ was measured for the proton $H_{ax}$ of CB[5]$OH_{10}$ in the presence and the absence of NaCl 9 g/l and of $O_2$ 1 bar at two different temperatures.

| $R_1$ $CB_5OH_{10}$ ($s^{-1}$) | Without $O_2$ | With $O_2$ |
|---|---|---|
| Without NaCl, 21°C | 2.2 | 18.5 |
| With NaCl, 37°C | 2.1 | 20.8 |

The slightly different temperature softly influences the $R_1$.

Conclusion: NaCl does not compete with $O_2$ for complexation.

**II-2. CB[5]**

Besides of the two doublets corresponding to $H_{ax}$ and $H_{eq}$ (ca. 5.69 ppm and 4.35 ppm respectively), the spectra of CB[5] displayed on figures 4a and 4b show a singlet at 5.5 ppm that corresponds to $R_1$ = H.

The fact that the spectra SP1 and SP2 are identical proves that $O_2$ is not significantly encapsulated by CB[5].

**II-3. CB[5]$Me_{10}$**

The characteristic signal of the methyl groups of CB[5]$Me_{10}$ which resonates at 1.8 ppm is not represented on the spectra displayed on figures 5a and 5b.

The comparison of spectra SP1, SP2 and SP3 shows that $O_2$ is encapsulated by CB[5]$Me_{10}$ in the absence of NaCl, but is no longer so significantly in the presence of NaCl. The main ions present in the blood are therefore too strong competitors of $O_2$ for the encapsulation in CB[5]$Me_{10}$.

**III - Conclusion**

Therefore, the results displayed above show that CB[5]$OH_{10}$ exhibits a remarkably strong affinity for oxygen, notably in comparison with CB or CB[5]$Me_{10}$.

**Claims**

**1.** A compound being CB[5]$OH_{10}$, corresponding to the following formula:

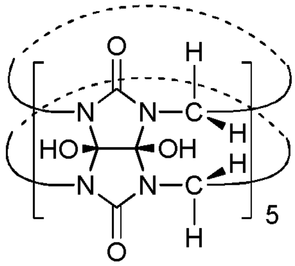

or a salt or a solvate thereof,
for therapeutic use as a physiological gas carrier, wherein said gas is $O_2$.

2. The compound for therapeutic use according to claim 1, wherein said compound is covalently linked to one or several hydrophilic polymeric side-chains, such as polyethyleneglycol (PEG) or hydroxyethyl starch (HES).

3. The compound for therapeutic use according to claim 1 or 2, wherein said compound is used as a hemoglobin substitute.

4. The compound for therapeutic use according to claim 1 or 2, for the treatment of ischemia.

5. The compound for therapeutic use according to claim 1 or 2, for the regulation of arterial blood pressure.

6. The compound for therapeutic use according to claim 1 or 2, for anti-ageing, skin protection or skin regeneration.

7. Non-therapeutic use of a compound being $CB[5]OH_{10}$, corresponding to the following formula:

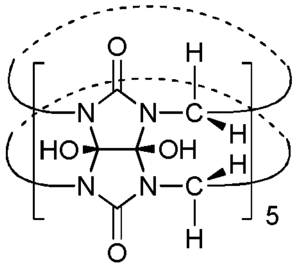

or a salt or a solvate thereof, for binding, carrying and/or releasing a gas, wherein said gas is $O_2$.

8. The non-therapeutic use according to claim 7, for the preservation and/or protection and/or regeneration of biological materials, such as cells, tissues, body fluids and organs in vitro, and of microorganisms.

9. A complex of a compound as defined in claim 1 comprising a gas encapsulated in its inner cavity, wherein said gas is $O_2$.

10. A cosmetic composition comprising at least one complex as defined in claim 9 and at least one cosmetically acceptable excipient.

**Patentansprüche**

1. Eine Verbindung mit der Bezeichnung $CB[5]OH_{10}$, die der folgenden Formel entspricht:

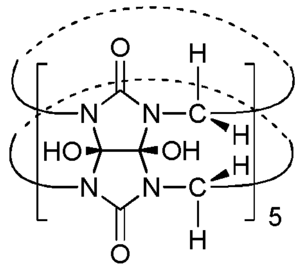

oder ein Salz oder ein Solvat davon,
zur therapeutischen Anwendung als physiologischer Gasträger, wobei das Gas $O_2$ ist.

2. Verbindung zur therapeutischen Anwendung nach Anspruch 1, wobei die Verbindung kovalent an eine oder mehrere hydrophile polymere Seitenketten, wie Polyethylenglykol (PEG) oder Hydroxyethylstärke (HES), gebunden ist.

3. Verbindung zur therapeutischen Anwendung nach Anspruch 1 oder 2, wobei die Verbindung als Hämoglobinersatz verwendet wird.

4. Verbindung zur therapeutischen Anwendung nach Anspruch 1 oder 2, zur Behandlung von Ischämie.

5. Verbindung zur therapeutischen Anwendung nach Anspruch 1 oder 2, zur Regulierung des arteriellen Blutdrucks.

6. Verbindung zur therapeutischen Anwendung nach Anspruch 1 oder 2, zum Anti-Aging, zum Hautschutz oder zur Hautregeneration.

7. Nichttherapeutische Anwendung einer Verbindung mit der Bezeichnung $CB[5]OH_{10}$, die der folgenden Formel entspricht:

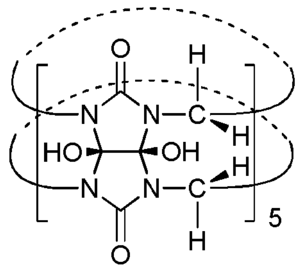

oder ein Salz oder ein Solvat davon,
zum Binden, Tragen und/oder Freisetzen eines Gases, wobei das Gas $O_2$ ist.

8. Nichttherapeutische Anwendung nach Anspruch 7, zur Erhaltung und/oder zum Schutz und/oder zur Regeneration biologischer Materialien, wie Zellen, Gewebe, Körperflüssigkeiten und Organe in vitro, und von Mikroorganismen.

9. Komplex einer Verbindung nach Anspruch 1, umfassend ein in seinem Innenhohlraum eingekapseltes Gas, wobei das Gas $O_2$ ist.

10. Kosmetische Zusammensetzung, umfassend wenigstens einen Komplex gemäß Anspruch 9 und wenigstens einen kosmetisch akzeptablen Hilfsstoff.

## Revendications

1. Composé qui est du $CB[5]OH_{10}$, correspondant à la formule suivante :

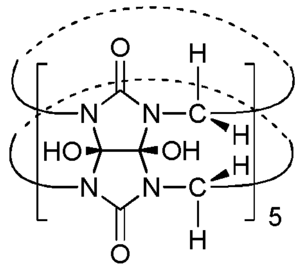

ou sel ou solvate de celui-ci,
pour une utilisation thérapeutique comme vecteur de gaz physiologique, dans lequel ledit gaz est de l'$O_2$.

2. Composé pour utilisation thérapeutique selon la revendication 1, dans lequel ledit composé est lié de manière covalente à une ou plusieurs chaînes latérales polymèriques hydrophiles, telles que le polyéthylène glycol (PEG) ou l'hydroxyéthylamidon (HES).

3. Composé pour utilisation thérapeutique selon la revendication 1 ou 2, dans lequel ledit composé est utilisé comme substitut d'hémoglobine.

4. Composé pour utilisation thérapeutique selon la revendication 1 ou 2, pour le traitement de l'ischémie.

5. Composé pour utilisation thérapeutique selon la revendication 1 ou 2, pour la régulation de la pression artérielle.

6. Composé pour utilisation thérapeutique selon la revendication 1 ou 2, pour l'anti-vieillissement, la protection de la peau ou la régénération de la peau.

7. Utilisation non thérapeutique d'un composé qui est du CB[5]$OH_{10}$, correspondant à la formule suivante :

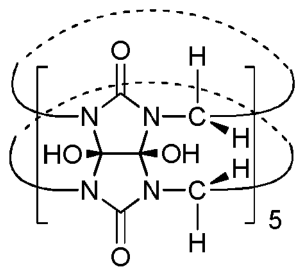

ou d'un sel ou d'un solvate de celui-ci,
pour lier, transporter et/ou libérer un gaz, ledit gaz étant de l'$O_2$.

8. Utilisation non thérapeutique selon la revendication 7, pour la conservation et/ou la protection et/ou la régénération de matériaux biologiques, tels que des cellules, des tissus, des fluides corporels et des organes in vitro, et de micro-organismes.

9. Complexe d'un composé selon la revendication 1 comprenant un gaz encapsulé dans sa cavité interne, dans lequel ledit gaz est de l'$O_2$.

10. Composition cosmétique comprenant au moins un complexe tel que défini dans la revendication 9 et au moins un excipient cosmétiquement acceptable.

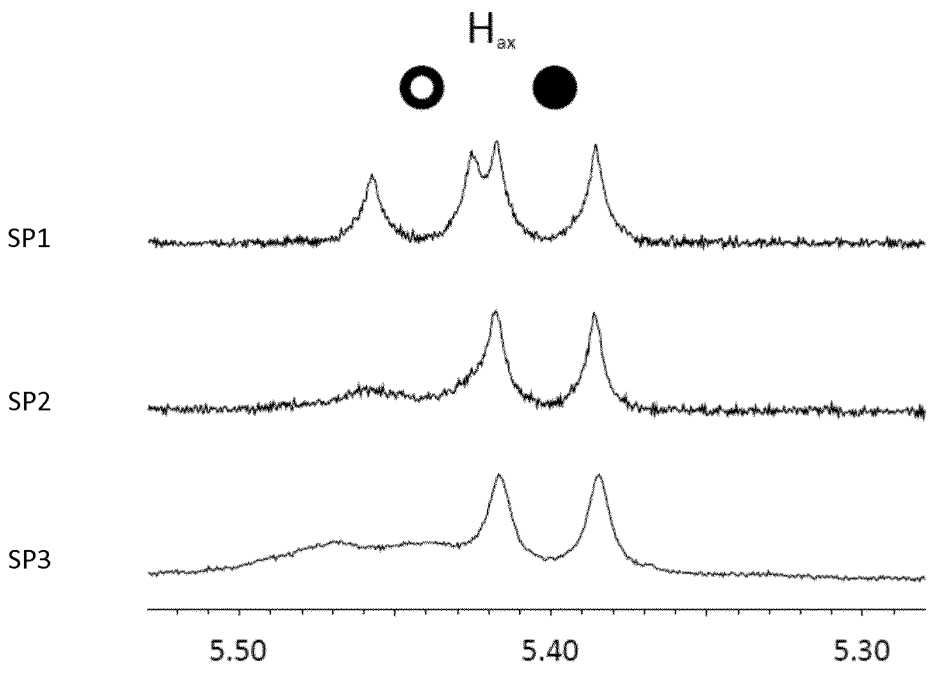


**Fig. 1a**

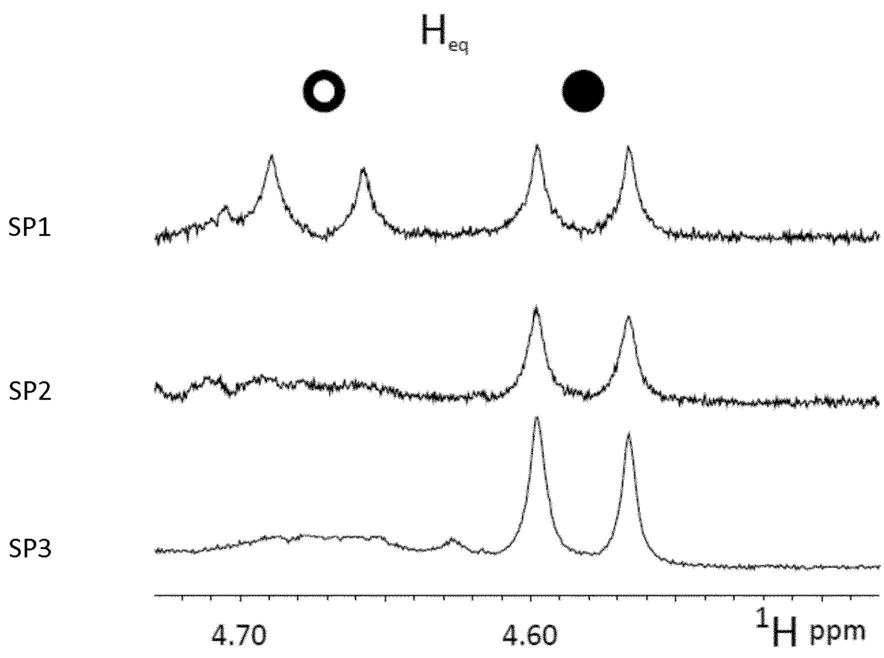


**Fig. 1b**

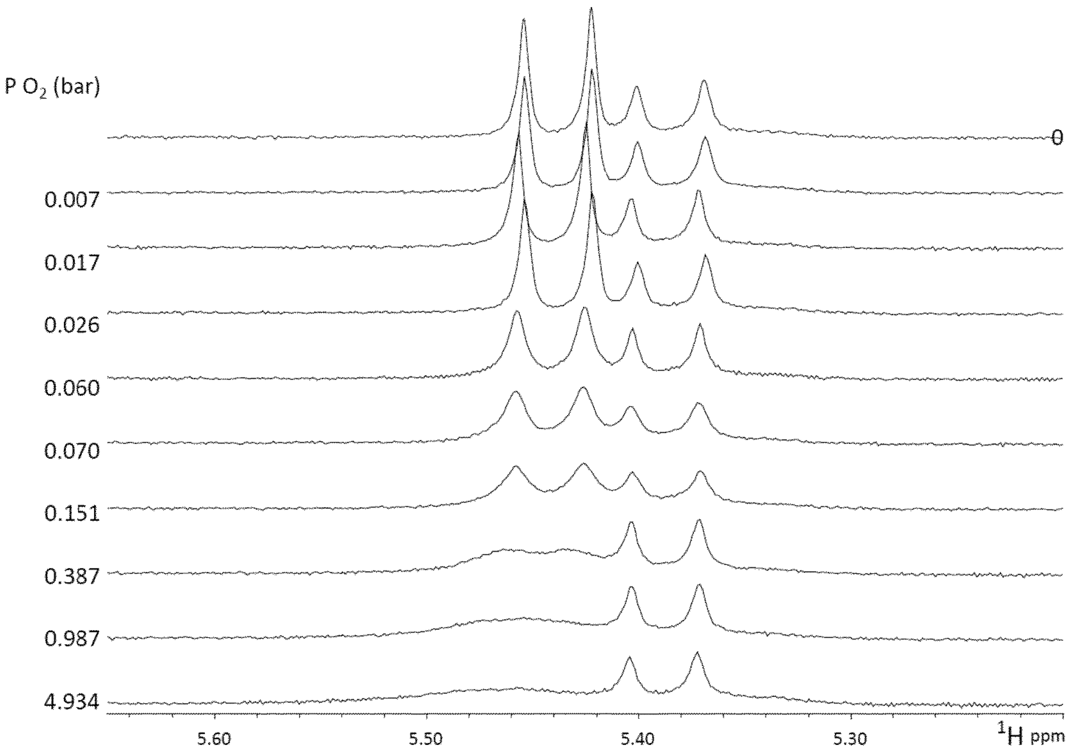
P $O_2$ (bar)
0
0.007
0.017
0.026
0.060
0.070
0.151
0.387
0.987
4.934
5.60
5.50
5.40
5.30
$^1$H ppm

Fig. 2

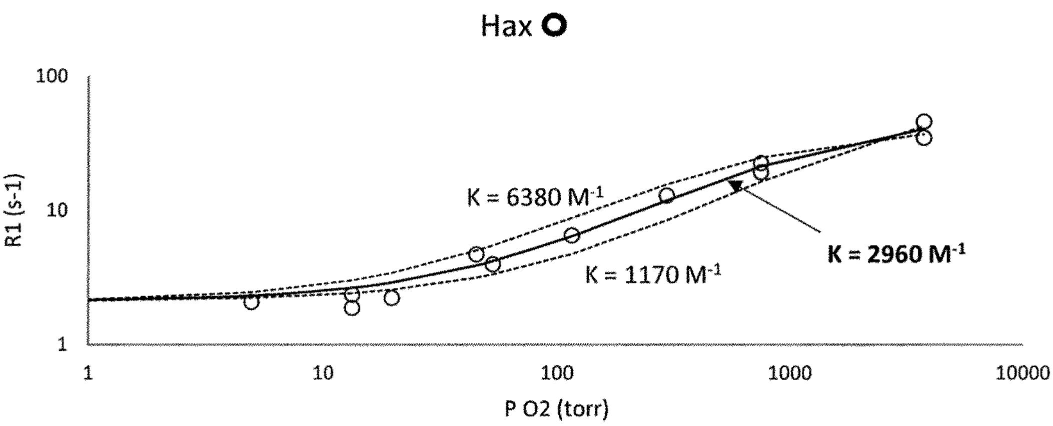
Hax
100
10
1
R1 (s-1)
K = 6380 M-1
K = 1170 M-1
K = 2960 M-1
1
10
100
1000
10000
P O2 (torr)

Fig. 3

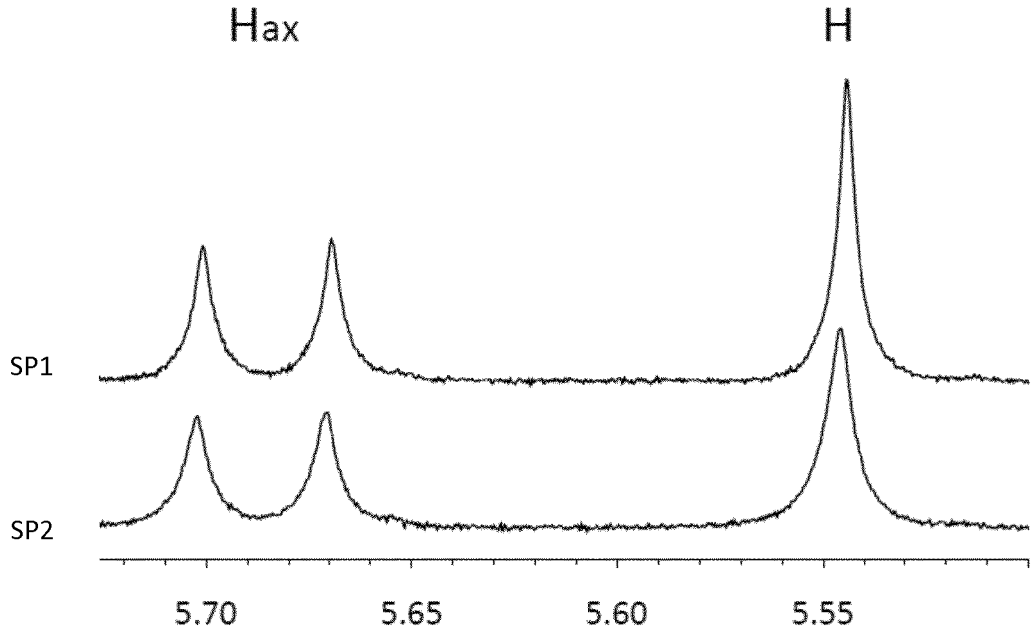


**Fig. 4a**

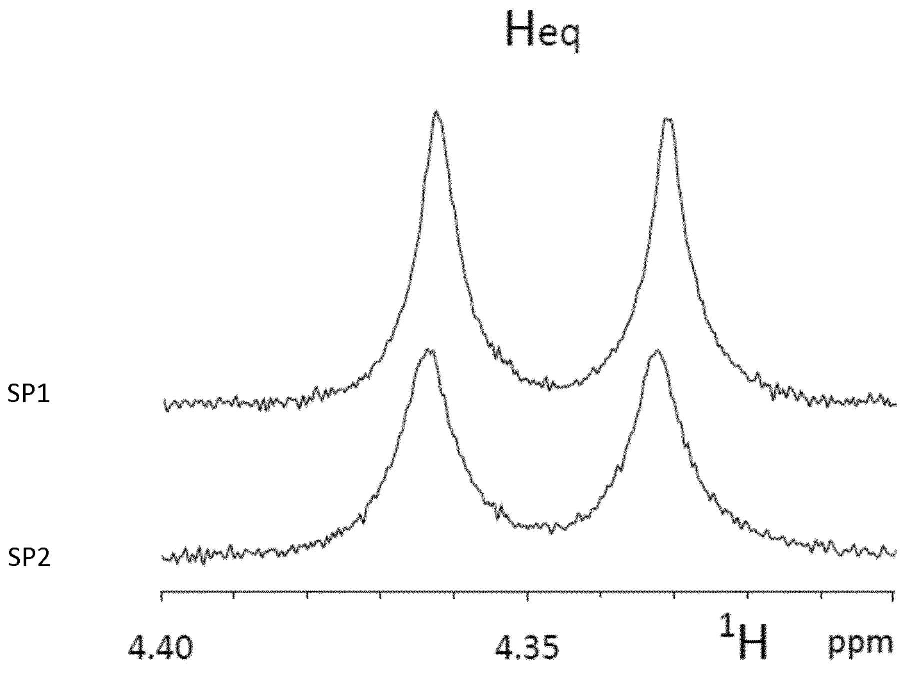


**Fig. 4b**

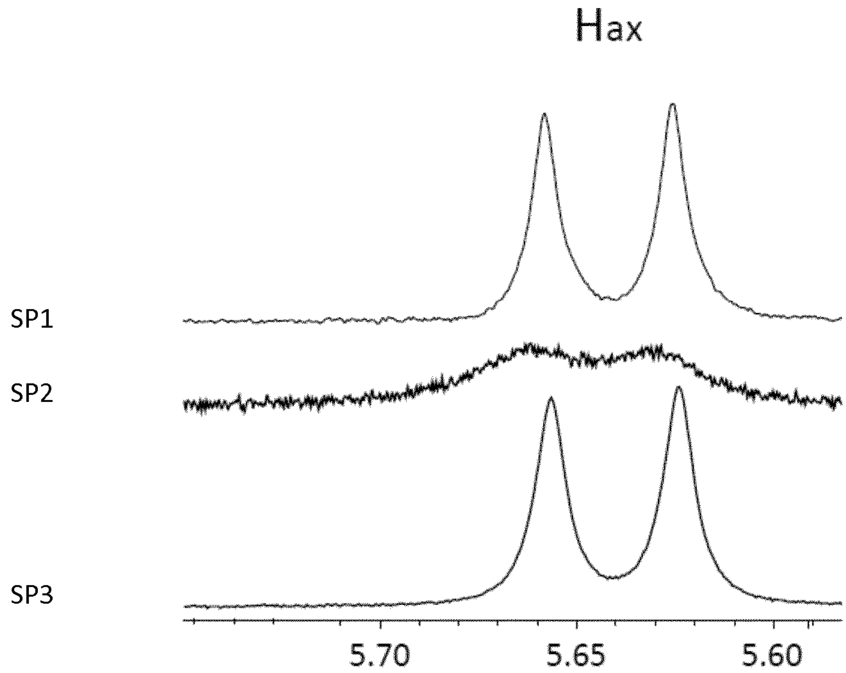


Fig. 5a

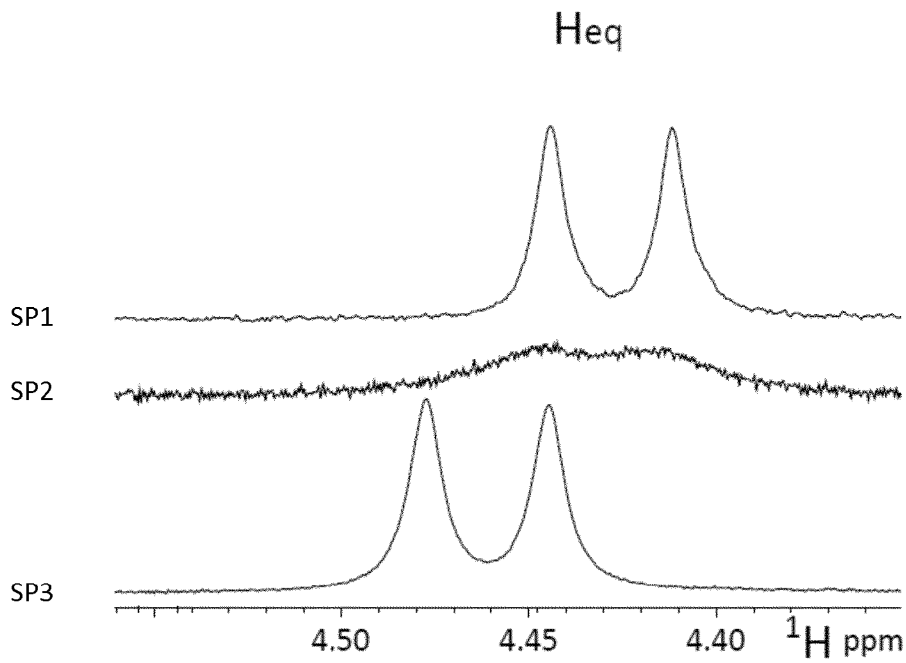


Fig. 5b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6793839 B, Day
- US 6869466 B
- WO 03055888 A
- WO 2010128159 A


### Non-patent literature cited in the description


- **BEHREND et al.** *Justus Liebigs Ann. Chem. Soc.,* 1905, vol. 339, 1
- **MOCK et al.** *J. Am. Chem. Soc.,* 1981, vol. 103, 7367-7368
- **NAU et al.** *Chem. Soc. Rev.,* 2015, vol. 44, 394-418
- International Cosmetic Ingredient Dictionary and Handbook. The Cosmetic, Toiletry, and Fragrance Assoc, 1997
- ICT Handbook. 1612-13
- ICT handbook. 1656-61
- ICT Handbook. 1639-40
- ICT Handbook. 1654-55
- *J. Am. Chem. Soc.,* 2003, vol. 125, 10186
- *Angew. Chem. Int. Ed,* 1992, vol. 31, 1475